# EUROPEAN PATENT APPLICATION

(11) **EP 4 818 792 A1**
(43) Date of publication of application: **07.10.2026**
(21) Application number: 24897443.8
(22) Date of filing: 21.11.2024
(51) Int. Cl.: G02B 27/02, A61B 3/113, A61B 5/16, A61B 5/347, A61B 5/349, A61B 5/398, G02B 26/08, H04N 5/64, H04N 13/128, H04N 13/344, H04N 13/383

(54) **VIDEO DISPLAY DEVICE, METHOD FOR DRIVING VIDEO DISPLAY DEVICE, AND PROGRAM THEREFOR**

(30) Priority: 29.11.2023 JP 2023201980
(71) Applicant: Canon Kabushiki Kaisha, Tokyo 146-8501 (JP)
(72) Inventor: MORITA, HIROMITSU, Tokyo 146-8501 (JP); ATSUTA, AKIO, Tokyo 146-8501 (JP)
(74) Representative: Canon Europe Limited
(86) International application number: PCT/JP2024/041335
(87) International publication number: WO 2025/115757

(57) **Abstract**

To solve the above-described problem, an image display apparatus according to the present application includes: first and second image display units configured to display first and second images for a right eye and a left eye of a user, respectively; first and second display optical elements corresponding to the first and second image display units, respectively; and an actuator configured to change positions of the display optical elements, wherein the actuator is driven based on a detection result of a physiological state or a psychological state of the user during use of the image display apparatus to change a position of at least one of the first and second display optical elements.

## Description

### Technical Field

The present invention relates to a technique for diopter adjustment in an image display apparatus.

### Background Art

There is an image display apparatus such as a head-mounted display that a user wears on the head for use, or an image display apparatus that a user wears like eyeglasses for use. A display unit is positioned near the eyes of the user, and display processing of a parallax image is performed for the respective left and right eyes of the user. By visually recognizing the displayed parallax image, the user obtains a stereoscopic effect for an object displayed in the parallax image.

In this image display apparatus, the line of sight of the user is adjusted to a position of a stereoscopic image generated based on images displayed for the respective left and right eyes, whereas a focal position is adjusted to each of the left and right images on a display screen. This results in an unnatural state that would not occur when viewing a real object.

For example, when the user views an image of an object displayed as a parallax image, the convergence angle of the user changes in accordance with the position of the displayed object. At this time, based on experience in real space, focal lengths of crystalline lenses change in accordance with the magnitude of the convergence angle. In this case, the focal lengths of the crystalline lenses and the dioptric power of the image display apparatus may be inconsistent, resulting in failure to ensure desired visibility, such as difficulty in clearly viewing the displayed parallax image due to the failure to achieve focus on the displayed parallax image. In the diopter adjustment of the image display apparatus, therefore, it is desirable to change the dioptric power in accordance with depth information in the parallax image that determines the convergence angle of the user, by using a method of driving a lens in the apparatus, for example.

PTL 1 enables a reduction in discomfort during stereoscopic viewing by performing diopter adjustment for depth information that corresponds to the position of a fixation point of a user during viewing of an image, in a manner that accommodates differences among individuals and a use state of an image display apparatus.

### Citation List

### Patent Literature

PTL 1: Japanese Patent Laid-Open No. 2023-32278

### Summary of Invention

### Technical Problem

However, even if diopter adjustment reduces discomfort during stereoscopic viewing, continuous image viewing for a long period of time may result in motion sickness or fatigue accumulating due to accumulation of changes in the user's physical condition, deviations in diopter adjustment, and the like. In particular, it is known that motion sickness or fatigue often occurs when an image in which an object displayed in a parallax image is displayed at a position on the near side of the user is viewed for a long period of time. A user of the image display apparatus has frequent opportunities for long-time viewing, such as games and live performances, and it is unpleasant for the user to experience motion sickness or fatigue.

Accordingly, an object of the present invention is to provide an image display apparatus capable of reducing a burden on a user during long-time image viewing while providing a stereoscopic image by parallax to the user.

### Solution to Problem

To achieve the above-described object, an image display apparatus includes:
a first image display unit configured to display a first image for a right eye of a user;
a second image display unit configured to display a second image for a left eye of the user;
a first display optical element corresponding to the first image display unit;
a second display optical element corresponding to the second image display unit; and
an actuator configured to change positions of the display optical elements, wherein
the image display apparatus further includes a state detection unit configured to detect a physiological state or a psychological state of the user during use of the image display apparatus, and the actuator is driven based on a detection result of the state detection unit to change at least one of a position of the first display optical element relative to the first image display unit and a position of the second display optical element relative to the second image display unit.

### Advantageous Effects of Invention

According to an image display apparatus of the present invention, a burden on a user during display of a stereoscopic image by parallax can be reduced in accordance with a state of motion sickness or fatigue in the user during long-time image viewing.

### Brief Description of Drawings

[Fig. 1] Fig. 1 is an explanatory diagram illustrating a schematic configuration of an image display apparatus according to a first embodiment.
[Fig. 2A] Fig. 2A is an explanatory diagram illustrating a state in which a lens position is changed in the image display apparatus.
[Fig. 2B] Fig. 2B is an explanatory diagram illustrating a state in which a lens position is changed in the image display apparatus.
[Fig. 3A] Fig. 3A is an explanatory diagram illustrating an example of a stereoscopic image display by parallax.
[Fig. 3B] Fig. 3B is an explanatory diagram illustrating an example of a stereoscopic image display by parallax.
[Fig. 3C] Fig. 3C is an explanatory diagram illustrating an example of a stereoscopic image display by parallax.
[Fig. 4A] Fig. 4A is an explanatory diagram illustrating a convergence angle of eyeballs and dioptric power when fixating on a target object.
[Fig. 4B] Fig. 4B is an explanatory diagram illustrating a convergence angle of eyeballs and dioptric power when fixating on a target object.
[Fig. 4C] Fig. 4C is an explanatory diagram illustrating a convergence angle of eyeballs and dioptric power when fixating on a target object.
[Fig. 5] Fig. 5 is a flowchart illustrating operation of the image display apparatus according to the first embodiment.
[Fig. 6] Fig. 6 is an explanatory diagram illustrating changes in physiological state or psychological state and lens position according to the first embodiment.
[Fig. 7] Fig. 7 is an explanatory diagram illustrating a modification of the schematic configuration of the image display apparatus according to the first embodiment.
[Fig. 8] Fig. 8 is an explanatory diagram illustrating changes in physiological state or psychological state and lens position in a modification of the image display apparatus according to the first embodiment.
[Fig. 9] Fig. 9 is a flowchart illustrating operation of an image display apparatus according to a second embodiment.
[Fig. 10] Fig. 10 is an explanatory diagram illustrating changes in physiological state or psychological state and maximum driving speed according to the second embodiment.
[Fig. 11] Fig. 11 is a flowchart illustrating operation of a modification of the image display apparatus according to the second embodiment.
[Fig. 12] Fig. 12 is an explanatory diagram illustrating changes in physiological state or psychological state and maximum driving speed in the modification of the image display apparatus according to the second embodiment.

### Description of Embodiments

Embodiments of the present invention will be described in detail hereinafter with reference to the accompanying drawings. In the embodiments, a head-mounted display will be described as an example of an image display apparatus capable of performing stereoscopic display via a plurality of display optical elements by causing a plurality of image display units to respectively display a pair of images having parallax.

An image display apparatus according to the present invention includes first and second image display units configured to display first and second images for a right eye and a left eye of a user, respectively. The image display apparatus further includes first and second display optical elements corresponding to the first and second image display units, respectively, and an actuator configured to change positions of the display optical elements. In the image display apparatus, further, the actuator is driven based on a detection result of a physiological state or a psychological state of the user during use of the image display apparatus to change at least one of a position of the first display optical element relative to the first image display unit and a position of the second display optical element relative to the second image display unit.

The following embodiments provide representative example configurations, and the present invention is not limited to the description of the embodiments. An image display apparatus, a method for controlling the image display apparatus, and a program therefor can be configured in a desired combination without departing from the spirit of the present invention.

### <First Embodiment>

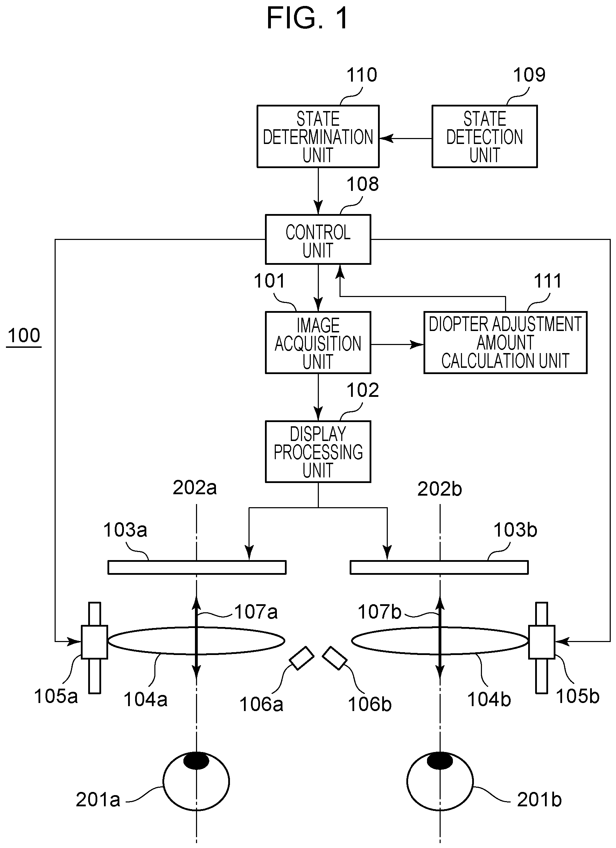

Fig. 1 is a diagram illustrating a schematic configuration of an image display apparatus 100 according to a first embodiment of the present invention.

The image display apparatus 100 includes an image acquisition unit 101 and a display processing unit 102. The image acquisition unit 100 acquires a display image to be displayed on an image display unit 103 through an external apparatus (not illustrated), a network, or the like. The display processing unit 102 performs display magnification adjustment processing and the like on the acquired image. The processed image is sent to and displayed on image display units 103a and 103b. For example, the image is divided for display on each of the image display units 103a and 103b; however, the configuration is not limited thereto. A single screen may be divided into two regions to display images corresponding to the divided image display units.

The image display apparatus 100 includes first and second display optical elements corresponding to a left eye 201a and a right eye 201b, respectively. For example, the first display optical element may have a lens 104a, and the second display optical element may have a lens 104b. Images displayed on the image display units 103a and 103b are respectively presented to the left eye 201a and the right eye 201b through the corresponding lenses 104a and 104b. Convex lenses illustrated in Fig. 1 may be used as the lenses.

The lenses 104a and 104b are driven by actuators 105a and 105b and are movable in directions along the optical axes of the lenses (arrows 107a and 107b). The present embodiment is described based on the assumption that optical axes 202a and 202b of the lenses 104a and 104b pass through the centers of the image display units 103a and 103b and the centers of the left eye 201a and the right eye 201b, respectively; however, it is not limited thereto.

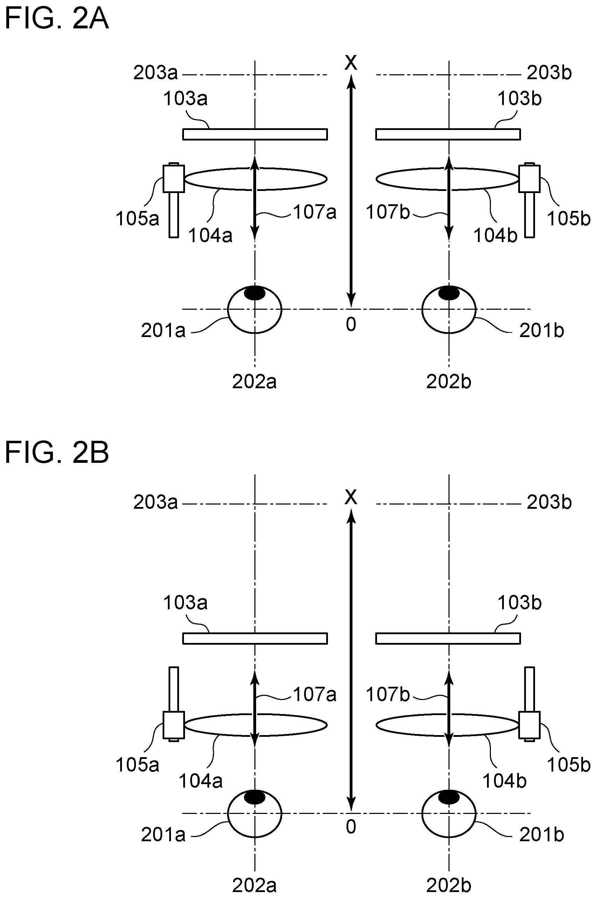

Figs. 2A and 2B are explanatory diagrams schematically illustrating states in which the lenses 104a and 104b are moved by the actuators 105a and 105b, respectively. Fig. 2A is a diagram illustrating a state in which the lenses 104a and 104b are moved to be close to the image display units 103a and 103b, respectively.

Fig. 2B is a diagram illustrating a state in which the lenses 104a and 104b are moved to be close to the left eye 201a and the right eye 201b, respectively. A user of the image display apparatus 100 visually recognizes virtual images 203a and 203b when viewing the image display units 103a and 103b with the left eye 201a and the right eye 201b through the lenses 104a and 104b. Here, the position of the virtual images 203a and 203b in directions of the optical axes 202a and 202b with reference to the position of the left eye 201a and the right eye 201b is defined as a virtual image formation position X. The virtual image formation position X can be changed by changing the position of the lenses 104a and 104b. For example, as illustrated in Fig. 2A, as the lenses 104a and 104b approach the image display units 103a and 103b, the virtual image formation position X moves closer to the left eye 201a and the right eye 201b. Conversely, as illustrated in Fig. 2B, as the lenses 104a and 104b approach the left eye 201a and the right eye 201b, the virtual image formation position X moves away from the left eye 201a and the right eye 201b. In this manner, moving the lenses 104a and 104b by the actuators 105a and 105b allows the dioptric power to be changed.

While the present embodiment describes a configuration in which, as the lenses 104a and 104b approach the image display units 103a and 103b, the virtual image formation position X moves closer to the left eye 201a and the right eye 201b, it is not limited thereto. In another configuration, as the lenses 104a and 104b approach the image display units 103a and 103b, the virtual image formation position X may move away from the left eye 201a and the right eye 201b. Further, in the above configuration, the lenses 104a and 104b are moved by the actuators 105a and 105b to change the dioptric power; however, the configuration is not limited to movement of an optical member. As a configuration alternative to the movement of the lenses 104a and 104b, an embodiment may be employed in which, for example, liquid lenses are adopted and the position of an interface formed of water and oil or the like is changed by an electrical signal applied from the actuators 105a and 105b. Further, since the image display apparatus 100, which is represented by a head-mounted display, is used while being positioned near the ears of the user, it is preferable to use a quiet actuator for changing the dioptric power, namely, an ultrasonic motor serving as a vibration actuator or an electromagnetic motor such as a voice coil motor. Further, while the lenses 104a and 104b are illustrated as single convex lenses in Figs. 1, 2A, and 2B, each of the lenses 104a and 104b may include a plurality of lenses configured such that the position of a predetermined lens is moved to change the dioptric power.

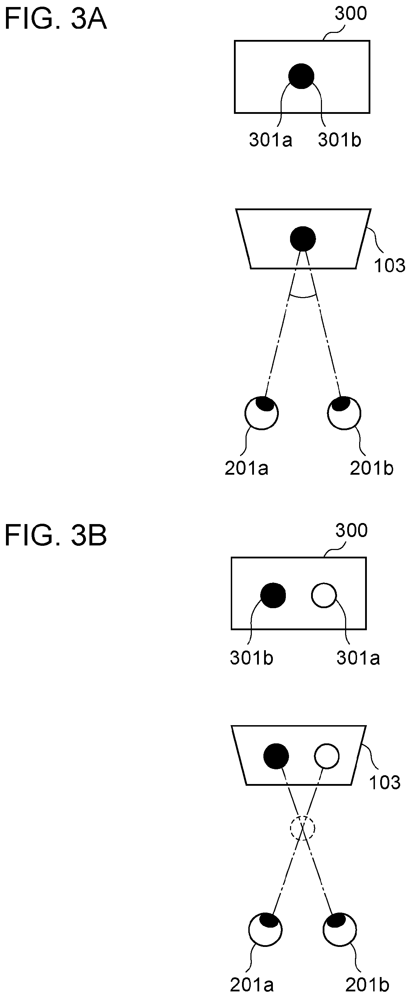

A parallax image 300 to be displayed on the image display units 103a and 103b will be described with reference to Figs. 3A to 3C. Figs. 3A to 3C are explanatory diagrams of an example of an image. The parallax image 300 includes, for example, a left-eye image 301a to be displayed on the image display unit 103a corresponding to the left eye 201a, and a right-eye image 301b to be displayed on the image display unit 103b corresponding to the right eye 201b.

While the present embodiment describes an example in which the parallax image 300 includes the left-eye image 301a and the right-eye image 301b, it is not limited thereto. For example, a parallax image to be displayed on each of the two image display units 103a and 103b may be generated by being processed by the display processing unit 102 based on three-dimensional data. Alternatively, an image captured by a camera mounted on the image display apparatus 100 may be handled as at least a portion of the parallax image 300. This configuration allows the image display apparatus 1 to display an image in an augmented reality space representation. For example, an image captured by a camera mounted on the image display apparatus 1 and an image based on three-dimensional data created in advance are superimposed on one another to create the parallax image 300. As a result, a composite image is displayed.

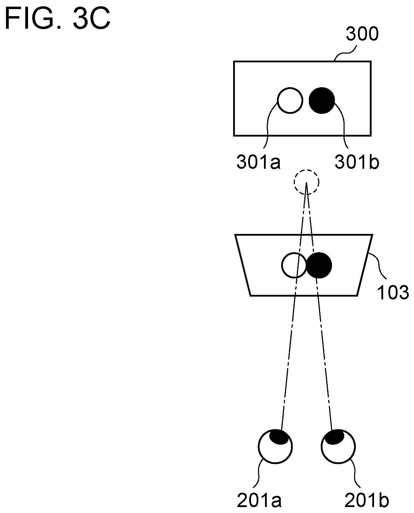

For example, as illustrated in Fig. 3A, when the lines of sight of the user converge at a position coinciding with the screen of a display image, the images 301a and 301b in the display image are perceived to be located at the same position as the image display unit 103. As illustrated in Fig. 3B, when the lines of sight of the user converge at a position in front of the image display unit 103, the images 301a and 301b in the display image are perceived to protrude relative to the image display unit 103. As illustrated in Fig. 3C, when the lines of sight of the user converge at a position behind the image display unit 103, the images 301a and 301b in the display image are perceived to be located far behind the screen relative to the image display unit 103.

While the present embodiment illustrates a case in which the images 301a and 301b are each a single image, it is not limited thereto. For example, a plurality of objects may be displayed in an image captured by a camera. In that case, gaze detection means 106a and 106b as illustrated in Fig. 1, which are capable of detecting gaze directions of the left eye 201a and the right eye 201b of the user, are mounted on the apparatus. Further, a process is performed in which a fixation point toward which the lines of sight of the user are directed is regarded as the images 301a and 301b. The gaze detection means may include an infrared illumination unit and a gaze detection camera, for example.

A diopter adjustment amount calculation unit 111 calculates a diopter adjustment amount based on depth information at the position of the fixation point determined from detection results by the gaze detection means 106a and 106b. Based on the calculated diopter adjustment amount, a control unit 108 outputs a drive command to drive the actuators 105a and 105b, thereby performing diopter adjustment. The respective spacings between the image display unit 103a and the lens 104a and between the image display unit 103b and the lens 104b are changed to adjust the dioptric power. For example, diopter adjustment is executed by the diopter detection means 106a and 106b in real time each time the fixation point of the user is changed.

The control unit 108 is a so-called microcomputer, and may include electrical components such as an arithmetic unit (CPU), a memory storing programs, and a memory as a work area onto which the programs are loaded. In that case, the control unit 108 serves to generate a signal having information for controlling driving of the actuators 105.

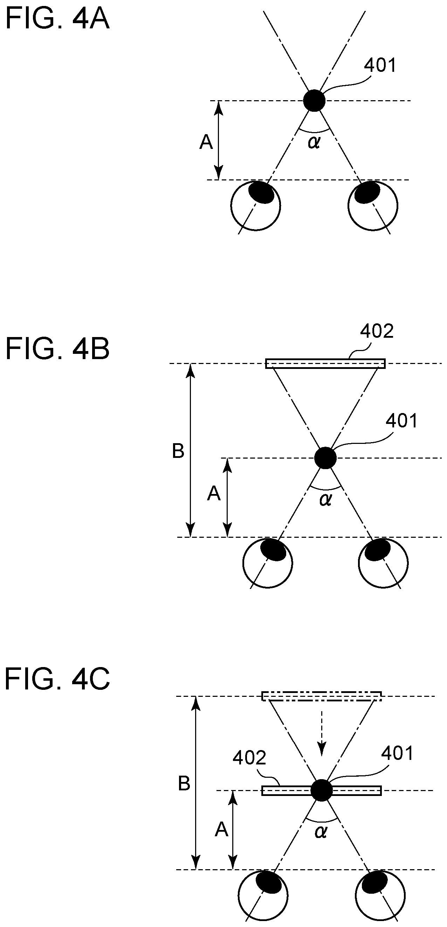

Figs. 4A to 4C are diagrams illustrating a convergence angle of eyeballs and dioptric power when fixating on a target object. Fig. 4A illustrates a state during fixation in the real world, and Fig. 4B illustrates a state during fixation in the image display apparatus 100.

In Fig. 4A, in the real world, the user is in a state of fixating on a target object 401 located at a position a distance A away. The line of sight of the left eye 201a of the user and the line of sight of the right eye 201b of the user converge on the target object 401 to form an angle α. The angle α corresponds to a convergence angle when the user fixates on the target object 401. In the real world, therefore, a distance corresponding to the convergence angle α and the distance A corresponding to the dioptric power are always consistent with each other.

On the other hand, in Fig. 4B, the user is in a state of fixating on an object 401 located at a position a distance A away in the image display apparatus 100. The convergence angle α is the same as that in the real world illustrated in Fig. 4A. A virtual image position 402 is a position of a virtual image formed when the user views the image display units 103a and 103b through the lenses 104a and 104b (not illustrated), respectively. The virtual image position 402 is a position located a distance B away from the left eye 201a and the right eye 201b.

Assigning the convergence angle α to the virtual image position 402 allows the user to visually recognize the virtual image as if it were present at the position of the target object 401. However, in the image display apparatus 100, unlike the real world, the distance corresponding to the convergence angle α and the distance B corresponding to the dioptric power are not consistent with each other. This is referred to as a vergence-accommodation conflict, and continuation of this state increases motion sickness or fatigue in the user.

Accordingly, as in Fig. 4C, changing the spacing between the image display units 103a and 103b and the lenses 104a and 104b (not illustrated) can change the virtual image position 402. For example, when it is detected by the gaze detection means that the user is fixating on the target object 401, the spacing between the display units and the lenses is changed such that the virtual image position 402 located at the distance B moves to the distance A. Thus, as illustrated in Fig. 4A, the distance corresponding to the convergence angle and the distance corresponding to the dioptric power are consistent with each other to provide a more natural state, thereby reducing motion sickness or fatigue.

To change the spacing between the display units and the lenses, it is sufficient to drive at least either the display units or the lenses.

A state detection unit 109 illustrated in Fig. 1 is configured to detect a physiological state or a psychological state of the user, and detects, for example, the heart rate, electrocardiogram, respiration, electrooculogram, skin potential, and center of gravity of the user.

As for the heart rate, the heart rate of the user is measured to determine the magnitudes of an average value of instantaneous heart rate, a respiratory component in heart rate variability, and a Mayer wave component in the heart rate variability, and at least one of these magnitudes is used as a detected value.

As for the electrocardiogram, the electrocardiogram of the user is measured to determine a high-frequency component (HF) and a low-frequency component (LF) of heart rate variability, which are magnitudes of specific frequency components of baseline fluctuation of the electrocardiogram, or the ratio of HF to LF, and at least one of these is used as a detected value.

As for respiration, the frequency, magnitude, and irregularity of respiration of the user are measured, and at least one of these is used as a detected value.

As for the electrooculogram, the blink count, cumulative frequency, cumulative count, rate of change, and blink interval of the user are measured, and at least one of these is used as a detected value.

Further, the center of gravity of the user may be measured, and the magnitude of sway of the center of gravity may be used as a detected value.

A detected value detected by the state detection unit 109 is output to a state determination unit 110 and is compared with a normal value recorded in advance, which is a predetermined value, to determine whether motion sickness or fatigue has occurred in the user. The physiological state or the psychological state may be determined by combining a plurality of factors among the electrocardiogram, the respiration, the electrooculogram, the skin potential, and the center of gravity, instead of using only one of these.

In a case where the skin potential is used, the determination may be performed based on the magnitude of a resistance component thereof. In a case where the center of gravity is used, the determination may be performed based on sway of the center of gravity.

If it is determined that the user has developed motion sickness or fatigue, the control unit 108 drives the actuators 105a and 105b to move the lenses 104a and 104b toward the image display units, thereby changing the dioptric power. The state detection unit 109 and the state determination unit 110 do not necessarily constitute part of the image display apparatus, and may be configured to wirelessly transmit and receive signals such that signals can be exchanged. Conversely, integrating the state detection unit 109 and the state determination unit 110 with the image display apparatus allows a user of the head-mounted display to immediately use the state detection function without the intervention of remote communication.

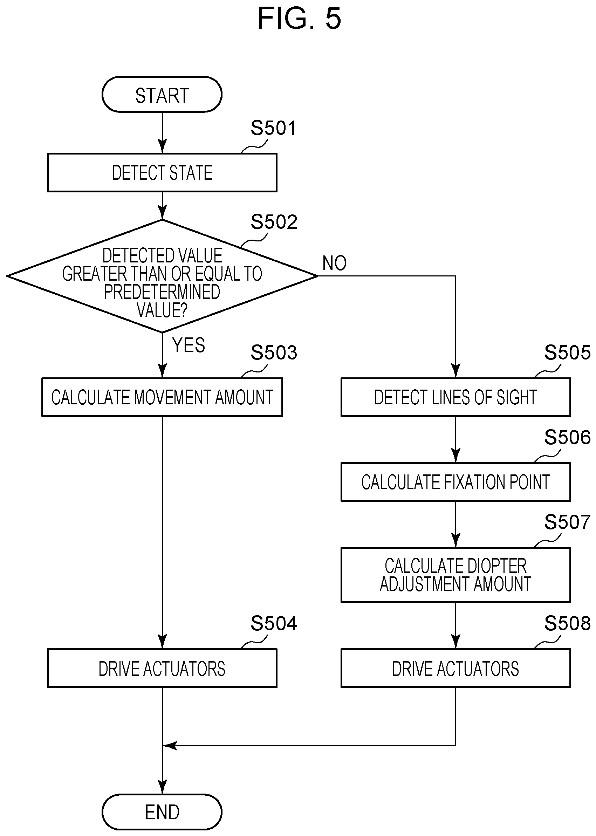

Operation according to the first embodiment will be described with reference to a flowchart illustrated in Fig. 5. A program corresponding to the flowchart illustrated in Fig. 5 is stored in a storage unit in an arithmetic processing unit. The control unit 108 includes a CPU, a memory, and the like, and each process illustrated in the flowchart of Fig. 5 is implemented by the CPU loading a predetermined program stored in the memory. The control unit 108 also may or may not be integrated with the image display apparatus.

First, in S501, a physiological state or a psychological state of the user is detected by using the state detection unit 109. Then, in S502, a state of motion sickness or fatigue of the user is determined based on the detected value. If the detected value is greater than or equal to a desired value, in S503, a movement amount is calculated so as to move the lenses 104a and 104b in a direction away from the image display units 103a and 103b, and in S504, the control unit 108 drives the actuators 105a and 105b. The movement amount may be determined in accordance with the detected value, or the movement amount may be increased as the detected value increases and may be decreased as the detected value decreases. This prevents the displayed parallax image from being in focus and being clearly viewed such that the amount of information that enters the left eye 201a and the right eye 201b is reduced to relax eyeball muscles, thereby reducing motion sickness or fatigue.

If the detected value is less than or equal to the desired value in S502, in S505, the gaze detection means 106a and 106b are used to detect the lines of sight of the left eye 201a and the right eye 201b of the user, respectively.

Then, in S506, the control unit 107 performs a process of determining a fixation point from the line of sight of the left eye 201a and the line of sight of the right eye 201b. The fixation point can be calculated by using an average value of the lines of sight of the left eye 201a and the right eye 201b.

Then, in S507, the diopter adjustment amount calculation unit 111 performs a process of calculating a diopter adjustment amount at the position of the fixation point of images to be displayed on the image display units 103a and 103b. Specifically, parallax between an image for the left eye at the fixation point position and an image for the right eye at the fixation point position is calculated, and a diopter adjustment amount is derived from depth information corresponding to the parallax. For example, table data in which parallax and a diopter adjustment amount are associated with each other is provided to calculate the diopter adjustment amount corresponding to the parallax. Alternatively, the diopter adjustment amount calculation unit 111 may calculate the diopter adjustment amount from the parallax by using a mathematical formula indicating a relationship between the parallax and the diopter adjustment amount.

Then, in S508, the control unit 108 drives the actuators 105a and 105b based on the diopter adjustment amount calculated by the diopter adjustment amount calculation unit 111. Thus, an operation of changing the spacing between the image display units 103a and 103b and the lenses 104a and 104b is performed.

Since the above operation is performed each time the fixation point is changed, diopter adjustment can be performed in real time to change the dioptric power.

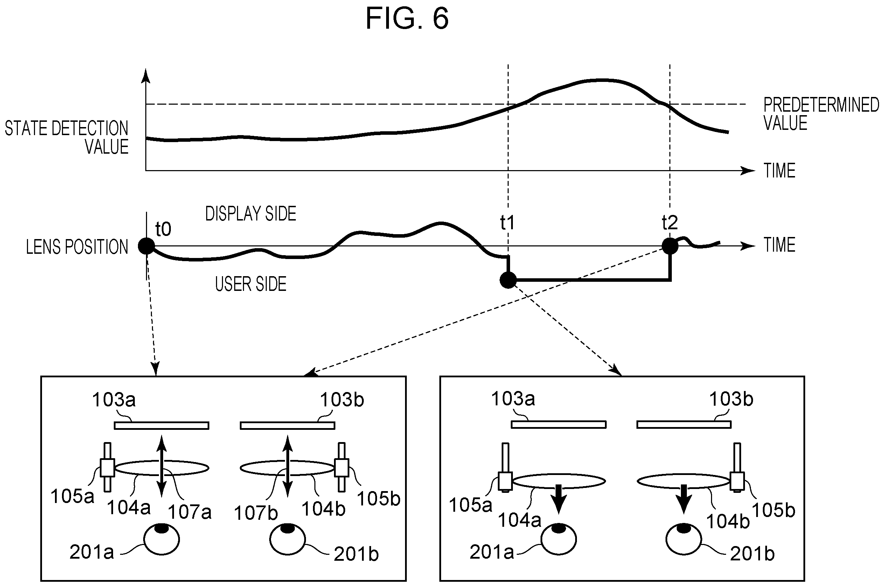

Fig. 6 illustrates detected values of a physiological state or a psychological state of the user and lens positions in the configuration illustrated in Fig. 1. As illustrated in Fig. 6, in a period from time t0 to t1, a fixation point is calculated from the lines of sight detected by the gaze detection means, and diopter adjustment is performed in real time in directions of the arrows 107a and 107b in accordance with depth information of the fixation point. When a state detection value detected by the state detection unit exceeds a predetermined value at time t1, the actuators 105a and 105b are operated to bring the lenses 104a and 104b closer to the user side such that the lenses 104a and 104b move away from the image display units 103a and 103b, respectively. Thereafter, when the detected value falls below the desired value at time t2, a fixation point is calculated again from the lines of sight detected by the gaze detection means, and diopter adjustment is performed in real time in the directions of the arrows 107a and 107b in accordance with depth information of the fixation point.

A configuration may be employed in which the reciprocal of the state detection value is set as an evaluation target and a case where the reciprocal of the state detection value falls below a certain value is used as a reference; this is synonymous with the state detection value "exceeding the predetermined value".

In the present example, since the lens 104a corresponding to the left eye 201a and the lens 104b corresponding to the right eye 201b can be driven by the independent actuators 105a and 105b, respectively, this can support even a user having different visual acuity in the left and right eyes. That is, a configuration can be adopted in which the position of at least one of the first and second display optical elements is changed.

According to the present example, diopter adjustment is performed in accordance with depth information of the fixation point of the lines of sight, and the actuators are driven so as to move the lenses 104a and 104b in a direction away from the image display units 103a and 103b in accordance with the state of motion sickness or fatigue in the user. This results in blurred perception of the images displayed on the image display units. Reducing the amount of information acquired by the user from the images can reduce the motion sickness or fatigue accumulated through long-time use.

While the above example describes operating the actuators such that the image display units and the lenses move away from each other, it is not limited thereto. The actuators may be operated such that the image display units and the lenses move closer to each other, and equivalent effects can be obtained. In this case, when the value detected by the state detection unit 109 exceeds the predetermined value, the lenses 104a and 104b are moved by the actuators 105a and 105b so as to be closer to the image display units 103a and 103b.

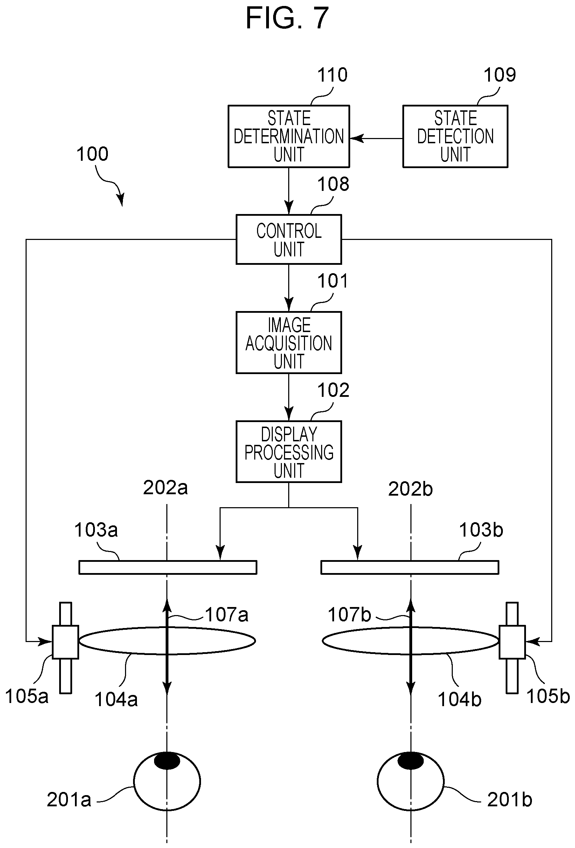

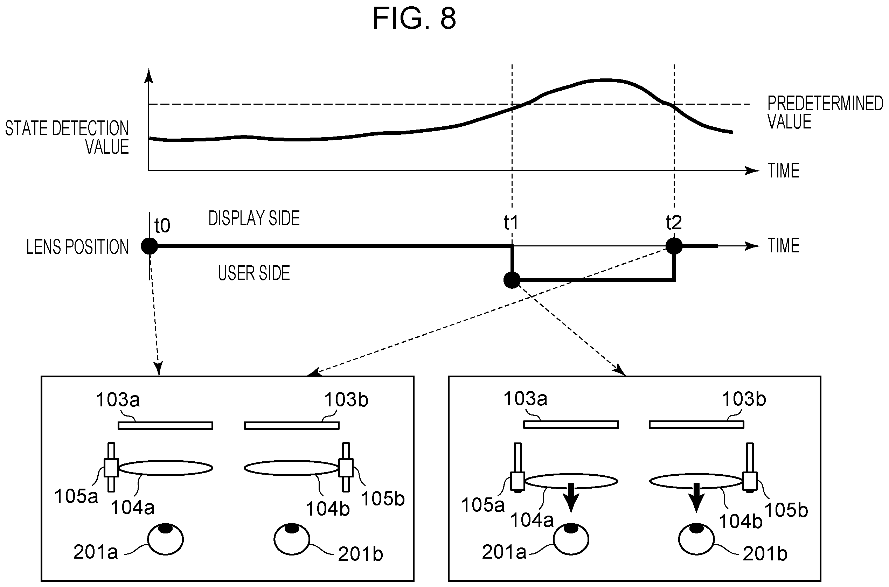

While the above description assumes a configuration for performing diopter adjustment based on depth information of the fixation point detected by the gaze detection means 106a and 106b, it is not limited thereto. A configuration as illustrated in Fig. 7 for manually performing diopter adjustment may be employed. Fig. 8 illustrates detected values of a physiological state or a psychological state of the user and lens positions in the configuration illustrated in Fig. 7. In this configuration, as illustrated in Fig. 8, at time t0, the user manually performs diopter adjustment to a position suited to a viewed image, and this position is set as a lens reference position. When the value detected by the state detection unit exceeds the predetermined value at time t1, the actuators are operated to bring the lenses closer to the user side such that the lenses move away from the display units. Thereafter, when the detected value falls below the desired value at time t2, the actuators are operated to return the lenses to the lens reference position manually adjusted at time t0.

### <Second Embodiment>

An operation method of the actuators 105a and 105b driven by the control unit 108 in the first embodiment described above using the values detected by the state detection unit 109 will be described. Since principles, configurations, and the like are similar to those of the first example, description thereof is omitted.

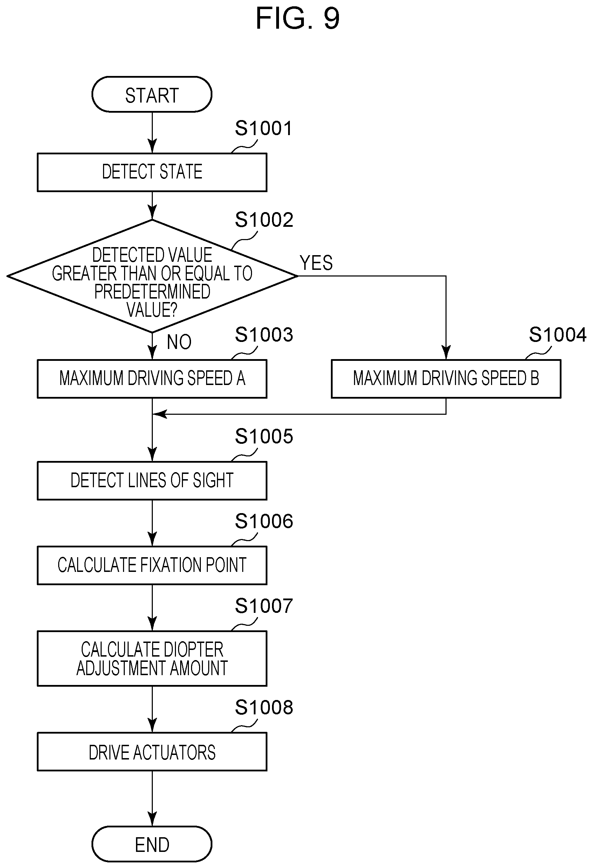

Operation according to the second embodiment will be described with reference to a flowchart illustrated in Fig. 9. A program corresponding to the flowchart illustrated in Fig. 9 is stored in a storage unit in an arithmetic processing unit. The control unit 108 includes a CPU, a memory, and the like, and each process illustrated in the flowchart of Fig. 9 is implemented by the CPU loading a predetermined program stored in the memory.

First, in S1001, a physiological state or a psychological state of the user is detected by using the state detection unit 109. Then, in S1002, a state of motion sickness or fatigue of the user is determined based on the detected value. If the detected value is less than or equal to a desired value, in S1003, the control unit 108 sets the maximum driving speeds of the actuators 105a and 105b to A (e.g., 100 mm/s). If the detected value is greater than or equal to the desired value, in S1004, the control unit 108 sets the maximum driving speeds of the actuators 105a and 105b to B (e.g., 50 mm/s) smaller than A. Changing the maximum driving speed in this manner can suppress the amount of change in the user's focus on the image, thereby reducing the tendency of the eyes to follow abrupt changes in focus. Thus, even during long-time image viewing, reducing the burden on the user can reduce the increase or accumulation of motion sickness or fatigue.

Then, in S1005, the gaze detection means 106a and 106b are used to detect the lines of sight of the left eye 201a and the right eye 201b of the user, respectively.

Then, in S1006, the control unit 108 performs a process of determining a fixation point from the line of sight of the left eye 201a and the line of sight of the right eye 201b. The fixation point can be calculated by using an average value of the lines of sight of the left eye 201a and the right eye 201b.

Then, in S1007, the diopter adjustment amount calculation unit 111 performs a process of calculating a diopter adjustment amount at the position of the fixation point of images to be displayed on the image display units 103a and 103b. Specifically, parallax between an image for the left eye at the fixation point position and an image for the right eye at the fixation point position is calculated, and a diopter adjustment amount is derived from depth information corresponding to the parallax. For example, table data in which parallax and a diopter adjustment amount are associated with each other is provided to calculate the diopter adjustment amount corresponding to the parallax. Alternatively, the diopter adjustment amount calculation unit 111 may calculate the diopter adjustment amount from the parallax by using a mathematical formula indicating a relationship between the parallax and the diopter adjustment amount.

Then, in S1008, the control unit 108 drives the actuators 105a and 105b based on the diopter adjustment amount calculated by the diopter adjustment amount calculation unit 111. Thus, an operation of changing the spacing between the image display units 103a and 103b and the lenses 104a and 104b is performed. Since the above operation is performed each time the fixation point is changed, diopter adjustment can be performed in real time to change the dioptric power.

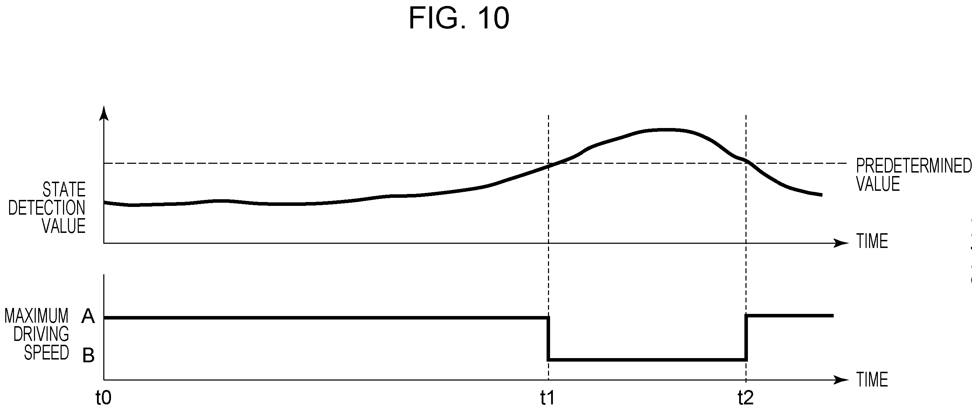

Fig. 10 illustrates detected values of a physiological state or a psychological state of the user and the maximum driving speeds of the actuators 105a and 105b. As illustrated in Fig. 10, the maximum driving speeds of the actuators 105a and 105b are set to A during a period from time t0 to t1, in which the values detected by the state detection unit 109 are less than the predetermined value. The maximum driving speed B, which is smaller than the maximum driving speed A, is set during a period from time t1 to t2, in which the values detected by the state detection unit 109 are greater than or equal to the predetermined value. The maximum driving speed A is set again during a period from time t2 to t3, in which the values detected by the state detection unit 109 are less than the predetermined value.

While the maximum driving speed is changed in this example, it is not limited thereto. A control gain or a control cycle may be changed, and in this case, similar effects can be obtained. The control gain refers to the ratio of control output required to change the actuator by a certain amount. Decreasing this ratio can suppress responsiveness and the amount of change in the user's focus on the image, thereby reducing the tendency of the eyes to follow abrupt changes in focus. Thus, even during long-time image viewing, reducing the burden on the user can reduce the increase or accumulation of motion sickness or fatigue. The control cycle refers to the time interval for issuing commands to control the actuator. Increasing this time interval can suppress the update frequency and the amount of change in the user's focus on the image, thereby reducing the tendency of the eyes to follow abrupt changes in focus. Thus, even during long-time image viewing, reducing the burden on the user can reduce the increase or accumulation of motion sickness or fatigue. Alternatively, if the value detected by the state detection unit 109 is greater than or equal to the predetermined value, two or more of the maximum driving speed, the control gain, and the control cycle may be simultaneously changed.

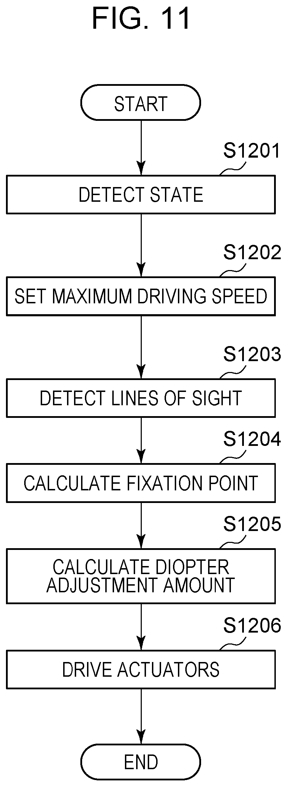

The above describes an example in which the maximum driving speed, the control gain, or the control cycle is changed in accordance with whether the value detected by the state detection unit 109 exceeds the predetermined value. However, the present invention is not limited to this example. As in a flowchart illustrated in Fig. 11, the maximum driving speed, the control gain, or the control cycle may be changed in accordance with the detected value. In this case, first, in S1201, a physiological state or a psychological state of the user is detected by using the state detection unit 109. Then, in S1202, the control unit 108 sets the maximum driving speeds of the actuators 105a and 105b, based on the detected value. For example, table data in which a detected value of a physiological state or a psychological state and a maximum driving speed are associated with each other is provided to calculate the maximum driving speed in accordance with the detected value of the physiological state or the psychological state. Alternatively, a mathematical formula indicating a relationship between a detected value of a physiological state or a psychological state and a maximum driving speed may be used to calculate a maximum driving speed. Changing the maximum driving speed in this manner can suppress the amount of change in the user's focus on the image, thereby reducing the tendency of the eyes to follow abrupt changes in focus. Thus, even during long-time image viewing, reducing the burden on the user can reduce the increase or accumulation of motion sickness or fatigue, thereby obtaining effects similar to those in the example described above.

Operations from S1203 to S1206 in Fig. 11 are similar to operations from S1005 to S1008 in the flowchart of Fig. 9 described above, and thus description thereof is omitted.

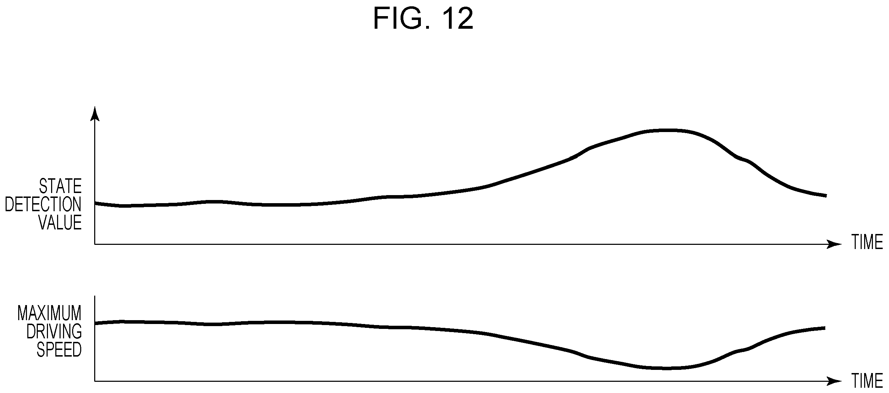

Fig. 12 illustrates detected values of a physiological state or a psychological state of the user and the maximum driving speeds of the actuators 105a and 105b. As illustrated in Fig. 12, the maximum driving speeds of the actuators 105a and 105b are set stepwise in accordance with the values detected by the state detection unit 109.

While the maximum driving speed is changed in this example, it is not limited thereto. A control gain or a control cycle may be changed, and in this case, similar effects can be obtained. Alternatively, if the value detected by the state detection unit 109 is greater than or equal to the predetermined value, two or more of the maximum driving speed, the control gain, and the control cycle may be simultaneously changed.

While the present invention has been described in detail with reference to preferred embodiments thereof, the present invention is not limited to these specific embodiments and may be modified in various ways without departing from the scope of the invention. Such modifications also fall within the scope of the present invention. A control method for executing the steps described above, and a non-transitory computer-readable program for executing the control method may also be provided.

The present invention may also be implemented by processing in which a program for implementing one or more functions of the embodiments described above is supplied to a system or an apparatus via a network or a storage medium, and one or more processors in a computer of the system or the apparatus read and execute the program. Further, the present invention may be implemented by circuitry (e.g., an ASIC) that implements one or more functions.

The present invention is not limited to the embodiments described above, and may be changed and modified in various ways without departing from the spirit and scope of the present invention. Accordingly, to apprise the public of the scope of the present invention, the following claims are made.

This application is based upon and claims the benefit of priority from Japanese Patent Application No. 2023-201980 filed November 29, 2023, the disclosure of which is incorporated herein in its entirety by reference.

### Reference Signs List

- 100: image display apparatus
- 101: image acquisition unit
- 102: display processing unit
- 103: image display unit
- 104: lens
- 105: actuator
- 106: gaze detection means
- 107: driving direction
- 108: control unit
- 109: state detection unit
- 110: state determination unit
- 111: depth information calculation unit
- 201a: left eye
- 201b: right eye
- 202: optical axis
- 203: virtual image

## Claims

1. An image display apparatus comprising:
a first image display unit configured to display a first image for a right eye of a user;
a second image display unit configured to display a second image for a left eye of the user;
a first display optical element corresponding to the first image display unit;
a second display optical element corresponding to the second image display unit; and
an actuator configured to change positions of the display optical elements, wherein
the image display apparatus further comprises a state detection unit configured to detect a physiological state or a psychological state of the user during use of the image display apparatus, and the actuator is driven based on a detection result of the state detection unit to change at least one of a position of the first display optical element relative to the first image display unit and a position of the second display optical element relative to the second image display unit.

2. The image display apparatus according to claim 1, wherein the actuator is driven such that at least one of a pair of the first image display unit and the first display optical element and a pair of the second image display unit and the second display optical element is positioned away from each other when the detection result of the physiological state or the psychological state is greater than or equal to a predetermined value.

3. The image display apparatus according to claim 1, wherein the actuator is driven such that at least one of a pair of the first image display unit and the first display optical element and a pair of the second image display unit and the second display optical element is positioned close to each other when the detection result of the physiological state or the psychological state is greater than or equal to a predetermined value.

4. The image display apparatus according to any one of claims 1 to 3, comprising first gaze detection means configured to perform gaze detection for the first image display unit, and second gaze detection means configured to perform gaze detection for the second image display unit.

5. The image display apparatus according to claim 4, wherein the gaze detection is performed without using the first display optical element and the second display optical element.

6. The image display apparatus according to any one of claims 1 to 3, further comprising calculation means configured to calculate a diopter adjustment amount corresponding to parallax between the first image and the second image at a position of a fixation point acquired from the first gaze detection means and the second gaze detection means.

7. The image display apparatus according to claim 6, comprising a control unit configured to output a drive command, wherein the control unit is configured to issue the drive command corresponding to the diopter adjustment amount to the actuator.

8. The image display apparatus according to any one of claims 1 to 3, wherein a control gain of the actuator is further changed in accordance with a value of the detection result of the physiological state or the psychological state.

9. The image display apparatus according to any one of claims 1 to 3, wherein a control cycle of the actuator is further changed in accordance with a value of the detection result of the physiological state or the psychological state.

10. The image display apparatus according to any one of claims 1 to 3, wherein a maximum driving speed of the actuator is further changed in accordance with a value of the detection result of the physiological state or the psychological state.

11. The image display apparatus according to claim 4, wherein the first gaze detection means and the second gaze detection means each include an infrared illumination unit and a gaze detection camera.

12. The image display apparatus according to any one of claims 1 to 3, wherein the state detection unit is configured to obtain the detection result of the physiological state or the psychological state, based on at least one of a blink count, a cumulative frequency, a rate of change, and a time interval between blinks that are derived from a measured electrooculogram of the user of the image display apparatus.

13. The image display apparatus according to any one of claims 1 to 3, wherein the state detection unit is configured to obtain the detection result of the physiological state or the psychological state, based on magnitudes or a ratio of frequency components of baseline fluctuation of a measured electrocardiogram of the user of the image display apparatus.

14. The image display apparatus according to any one of claims 1 to 3, wherein the state detection unit is configured to obtain the detection result of the physiological state or the psychological state, based on a magnitude of a resistance component of a measured skin potential of the user of the image display apparatus.

15. The image display apparatus according to any one of claims 1 to 3, wherein the state detection unit is configured to obtain the detection result of the physiological state or the psychological state, based on at least one of a measured frequency, magnitude, and irregularity of respiration of the user of the image display apparatus.

16. The image display apparatus according to any one of claims 1 to 3, wherein the state detection unit is configured to obtain the detection result of the physiological state or the psychological state, based on measured sway of a center of gravity of the user of the image display apparatus.

17. The image display apparatus according to any one of claims 1 to 3, wherein the state detection unit is configured to measure a measured heart rate of the user of the image display apparatus and obtain the detection result of the physiological state or the psychological state, based on an average value of instantaneous heart rate, a respiratory component in heart rate variability, and a Mayer wave component in the heart rate variability.

18. The image display apparatus according to any one of claims 1 to 3, wherein the first display optical element and the second display optical element are lenses.

19. The image display apparatus according to any one of claims 1 to 3, wherein the actuator is an electromagnetic motor or a vibration actuator.

20. A method for controlling an image display apparatus including:
a first image display unit configured to display a first image for a right eye of a user;
a second image display unit configured to display a second image for a left eye of the user;
a first display optical element corresponding to the first image display unit;
a second display optical element corresponding to the second image display unit; and
an actuator configured to change positions of the display optical elements,
the method comprising the steps of:
obtaining, by a control unit, a detection result of a physiological state or a psychological state of the user during use of the image display apparatus; and
driving, by the control unit, the actuator based on the detection result to change a position of at least one of the first display optical element and the second display optical element.

21. A non-transitory computer-readable program for causing the control unit to execute the steps of claim 20.
